# EUROPEAN PATENT SPECIFICATION

(11) **EP 0 366 076 B1**
(45) Date of publication and mention of the grant of the patent: **10.09.1997**
(21) Application number: 89119734.5
(22) Date of filing: 24.10.1989
(51) Int. Cl.: G06F 17/30

(54) **Method and apparatus for processing data in a medical information communicating system**
Verfahren und Vorrichtung zur Datenverarbeitung in einem Ubermittlungssystem für medizinische Informationen
Méthode et appareil pour le traitement des données dans un système de communication d'information en milieu médical

(30) Priority: 28.10.1988 JP 272439/88
(43) Date of publication of application: 02.05.1990
(73) Proprietor: KABUSHIKI KAISHA TOSHIBA, Kawasaki-shi, Kanagawa-ken 210 (JP)
(72) Inventor: Ema, Takehiro c/o Intellectual Property Division, Minato-ku Tokyo 105 (JP)
(74) Representative: Blumbach, Kramer & Partner

(56) References cited:
- GB-A- 2 164 771
- US-A- 4 604 653
- US-A- 4 672 683
- CRC CRITICAL REVIEWS IN DIAGNOSTIC IMAGING vol. 25, no. 4, 1986, C.RC. Press, CLEVELAND, OHIO, US pages 333 - 371; G.G.COX ET AL: 'COMPUTER NETWORKS FOR IMAGE MANAGEMENT IN RADIOLOGY: AN OVERVIEW'
- BIOMEDIZINISCHE TECHNIK vol. 31, no. 6, June 1986, FACHVERLAG SCHIELE UND SCHOEN,BERLIN pages 143 - 149; M.U.LEMKE ET AL.: 'WORK STATIONS FOR COMPUTER- GRAPHIC DISPLAY IN MEDICAL IMAGING'

## Description

The present invention relates to a method and apparatus for processing data in a medical information communicating system.

A PACS (Picture Archiving and Communication System) is constructed by modalities, network interface units, a star coupler, work stations, a data base or the like.

Each modality is constituted by a medical diagnosis apparatus, such as an X-ray diagnosis apparatus, MRI (Magnetic Resonance Imaging) apparatus or ultrasonic diagnosis apparatus. Image data of a patient acquired by each modality is stored in a data base. This image data is transferred via a star network constructed by a star coupler or the like. Each work station comprises a display and a console. A reading operation for image data specified through the console is executed by a doctor, a radiologist or the like. The reading operation represents a diagnosis operation for the image data of a patient.

At a reading operation, retrieval data is input via the console and an examination list yet to be read (hereinafter referred to as an unread examination list) is displayed on the display. The unread examination list is constituted by image addition data concerning image data yet to be read (hereinafter referred to as unread image data). The image addition data about a plurality of patients is written on this examination list. By referring to the examination list, a doctor inputs an image number for specifying image data and performs a diagnosis by displaying image data corresponding to the input image number on the display.

In referring to previous image data (hereinafter referred to as read image data), a patient previous examination list (hereinafter referred to as a read examination list) for a desired patient is displayed on the display by inputting a patient ID code and retrieval data. The read examination list is constituted by image addition data concerning the read image data. By inputting an image number, corresponding image data is displayed on the display. Accordingly, referring to the read image data, a reading operation for unread image data is performed.

For a data base, an optical disk is used as a long-term storage medium for image data. On the other hand, a magnetic disk or an IC (Integrated Circuit) memory is used as a short-term or temporary storage medium. With a work station, it is desirable to promptly display an image upon request, so that a magnetic disk and/or an IC memory is used as a storage medium.

Such a magnetic disk and IC memory cannot be used without limit. Further, it is not possible to determine in advance how many read image data of a patient are to be stored. Therefore, it is necessary to set the priority order for displaying image data. In other words, it is necessary to transfer in advance image data to be displayed soon from a data base to a work station by the priority order.

In displaying a read examination list, it is desirable to arrange in order from an image ID code corresponding to image data having a high probability for selection. This is because there is no guarantee that the entire examination list for a desired patient can be displayed on a limited display screen.

Further, in a continuous reading operation for unread image data of a plurality of patients, an unread examination list is produced in a data base in advance, and unread image data and the like are automatically prepared for transfer, thus reducing or eliminating the operation of image selection by a doctor.

The document US-A 4,672,683 discloses an image retrieval and storage system which comprises image storage systems, mixing means for mixing image signals out of said storage means, display means for displaying the image formed by said mixing means and indicating means for indicating a position of a part within the schematic image displayed on said display means. A data image is retrieved and displayed when a position on the schematic image is indicated by a light pen. No hint can be found in said document with respect to an arrangement of list data of a read examination list in accordance with a desired priority.

In view of the above, there is a demand for an apparatus which can improve the efficiency of a reading operation by doctors.

It is an object of the present invention to provide a method and apparatus for processing data in a medical information communicating system.

According to one aspect of the present invention, there is provided a method for processing data in a medical information communicating system as defined in claim 1.

According to another aspect of the present invention, there is provided an apparatus for processing data in a medical information communicating system as defined in claim 2.

This invention can be more fully understood from the following detailed description when taken in conjunction with the accompanying drawings, in which:
Fig. 1 is a block diagram illustrating the arrangement of a PACS;
Fig. 2 is a block diagram illustrating a work station in a PACS;
Fig. 3 is a block diagram illustrating a data base in a PACS;
Fig. 4 is a flowchart of a reading operation preparing process in a data base;
Fig. 5 is a flowchart of a reading operation process in a work station;
Fig. 6 is a flowchart of a reading operation result storing process in a data base;
Fig. 7 is a diagram illustrating the contents of image addition data;
Fig. 8 is a diagram illustrating the contents of image directory data;
Fig. 9 is a diagram illustrating the contents of an unread examination list;
Figs. 10A to 10C are diagrams illustrating the classification of unread examination lists;
Fig. 11 is a diagram illustrating the contents of a read examination list;
Fig. 12 is a diagram illustrating image addition data for every doctor ID code; and
Fig. 13 is a display example illustrating an examination list of a patient in a work station.

A preferred embodiment of the present invention will now be described referring to the accompanying drawings.

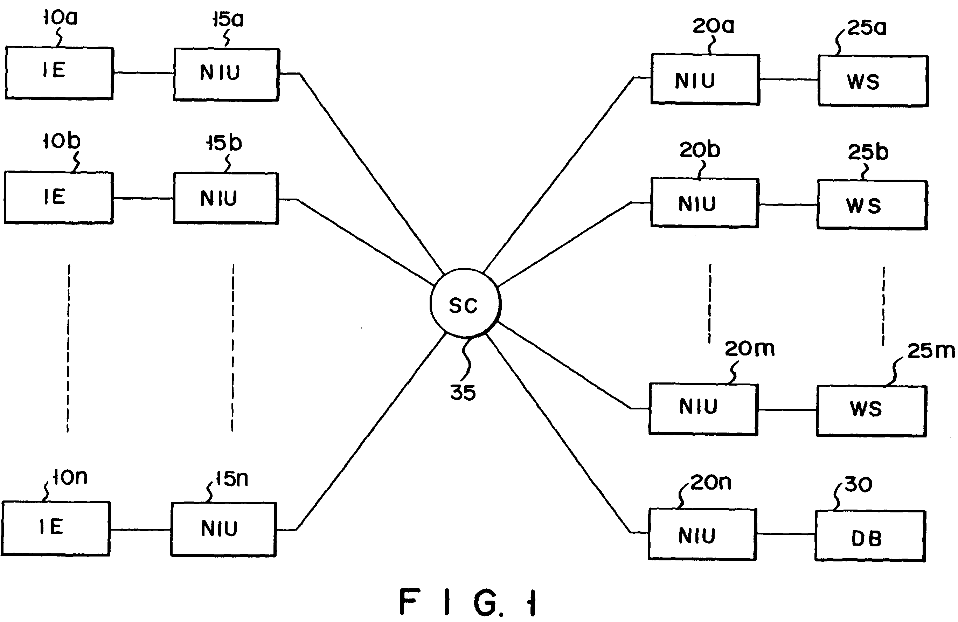

As shown in Fig. 1, a PACS according to this embodiment comprises modalities (IEs) 10a, 10b, ..., 10n, network interface units (NIUs) 15a, 15b, ..., 15n, 20a, 20b, ..., 20m, 20n, a star coupler (SC) 35, work stations (WSs) 25a, 25b, ..., 25m, and a data base (DB) 30.

The IEs 10a to 10n are constituted by medical diagnosis apparatus (modality) such as an X-ray diagnosis apparatus, an MRI apparatus or an ultrasonic diagnosis apparatus. Image data acquired by each apparatus is stored in the DB 30. Image data is transferred via a star network constructed by the SC 35, etc. The WSs 25a to 25m each comprise a display and a console, and a reading operation is performed on each work station. The reading operation represents a diagnosis operation for the image data of a patient.

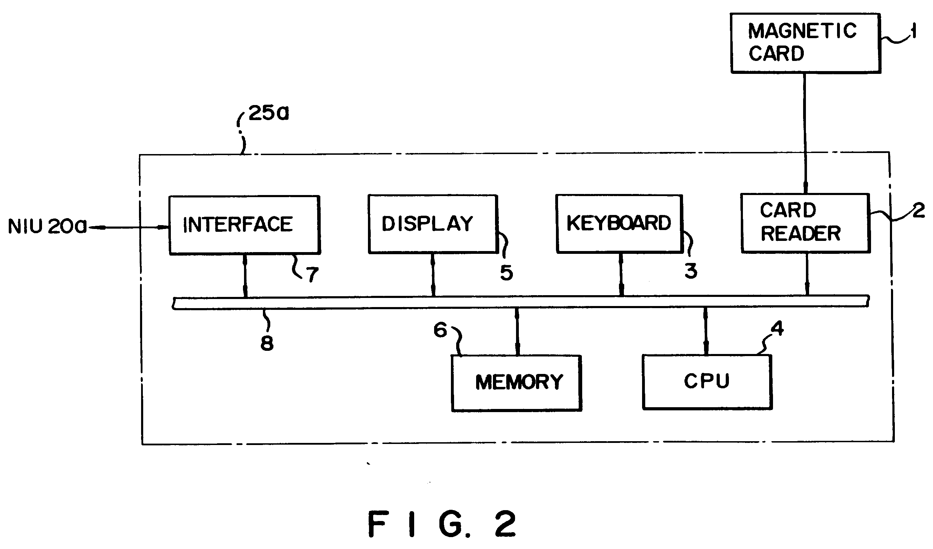

As shown in Fig. 2, the WS 25a comprises a card reader 2 for reading data stored in a magnetic card (MC) 1 serving as a portable recording medium, a keyboard 3, a memory 6, a CPU (Central Processing Unit) 4, a display 5, an interface 7 and a bus 8.

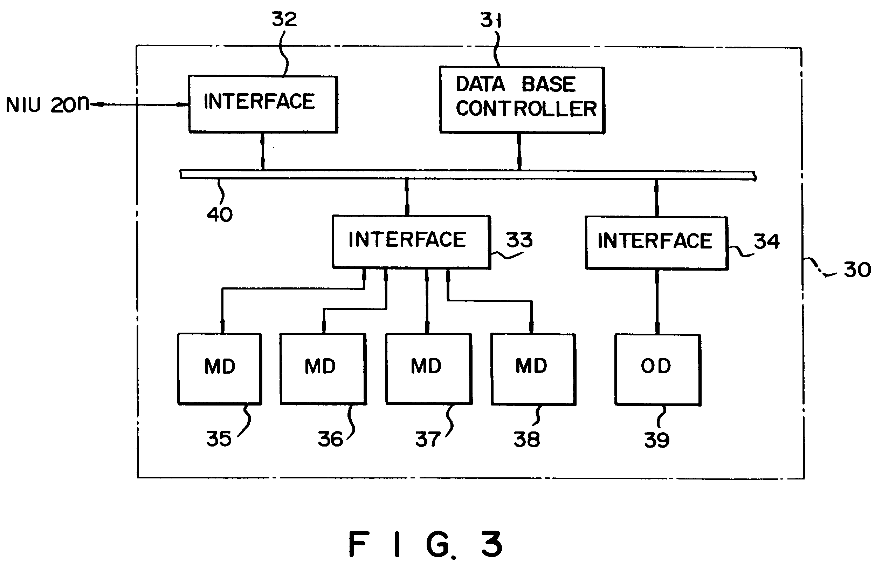

As shown in Fig. 3, the DB 30 is constructed by an optical disk (OD) 39 serving as a main storage, magnetic disks (MDs) 35 to 38 serving as auxiliary storages, a data base controller 31, interfaces 32 to 34 and a bus 40. In accordance with request by a work station, the data base controller 31 controls data transfer between the work station and the OD 39 or the MDs 35 to 38, and the operation of the OD 39 and MDs 35 to 38.

The MD 35 stores directory data and image addition data, the MD 36 an examination list, the MD 37 a reading report, and the MD 38 image data.

The operation of present system will be described below.

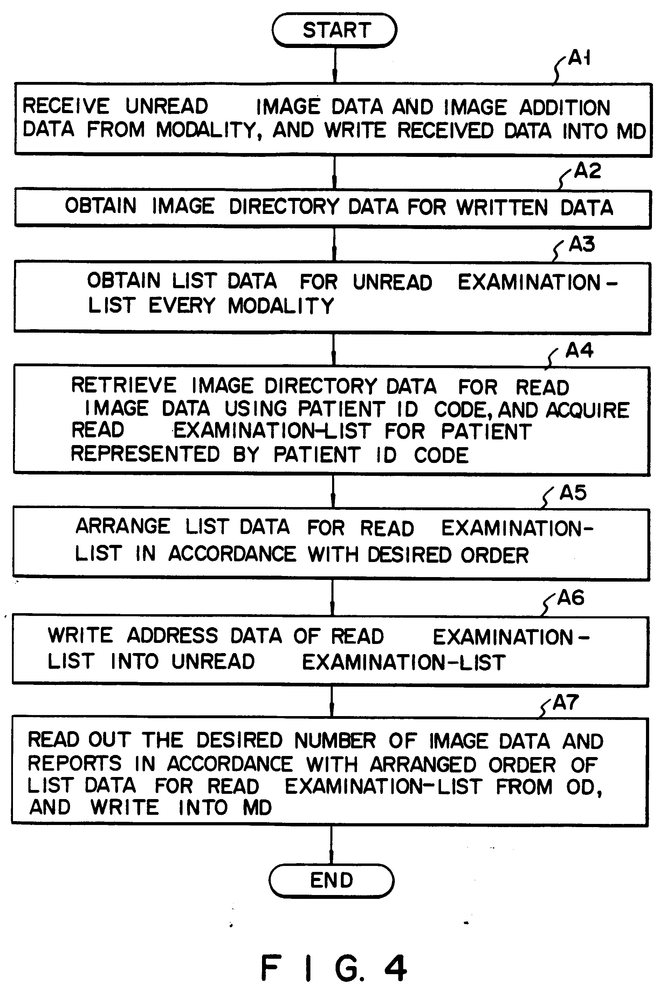

In the following description, the flowcharts shown in Figs. 4 to 6 will be referred to for explaining a reading operation preparing process in a data base, a reading operation process in a work station and a reading operation result storing process in the data base.

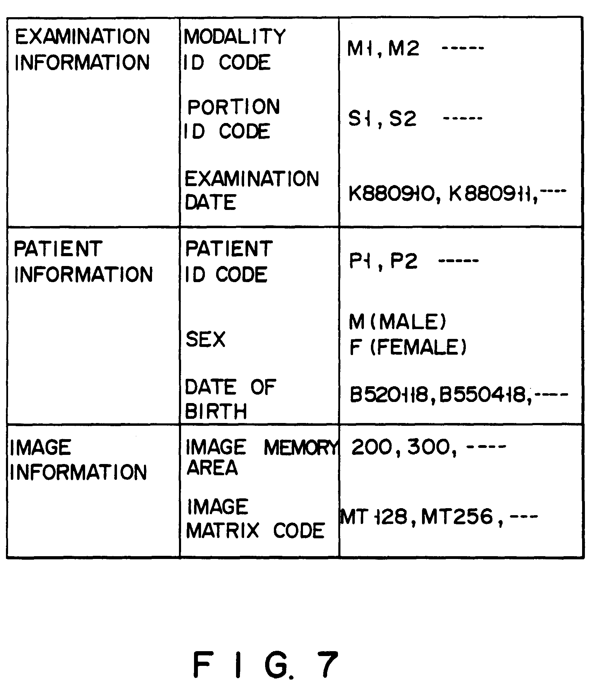

In the reading operation preparing process in the data base, as shown in Fig. 4, image data and image addition data transferred from a modality are received and written in MDs in step A1. The contents of the image addition data is illustrated in Fig. 7. The image addition data includes information such as an image ID code and a doctor ID code in addition to those shown in Fig. 7.

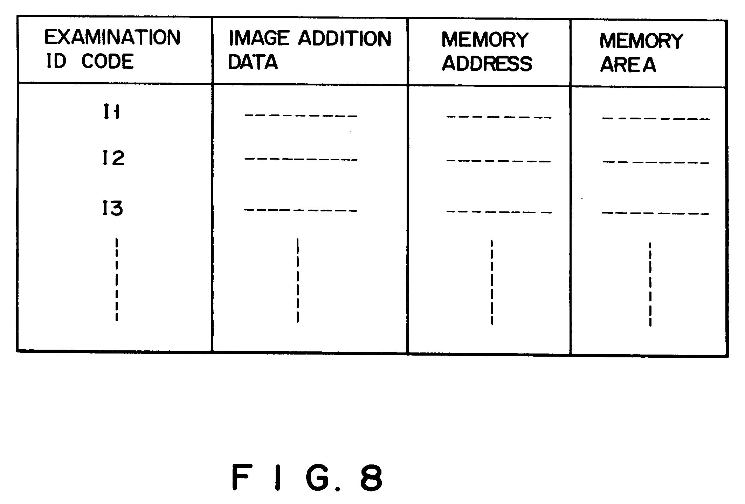

In step A2, image directory data for data written in the MD is obtained; this image directory data is additionally stored in a directory data storing MD. As shown in Fig. 8, the image directory data includes an examination ID code, image addition data, data representing a memory address and data representing a memory area.

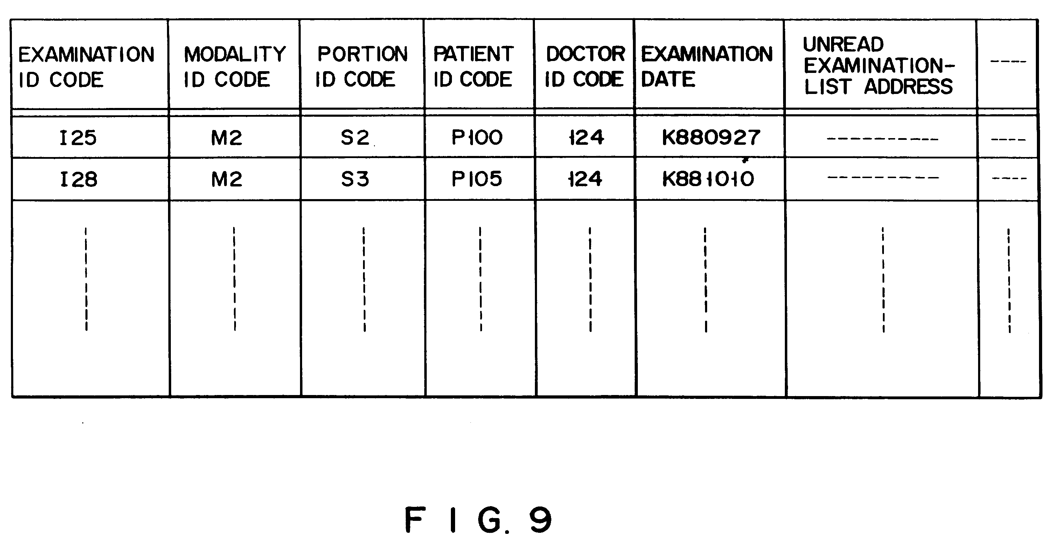

In step A3, list data for an examination list yet to be read (hereinafter referred to as an unread examination list) is obtained for each modality (see Fig. 9). This list data includes some information of the image addition data. The unread examination list is constituted by image addition data concerning image data yet to be read (hereinafter referred to as unread image data).

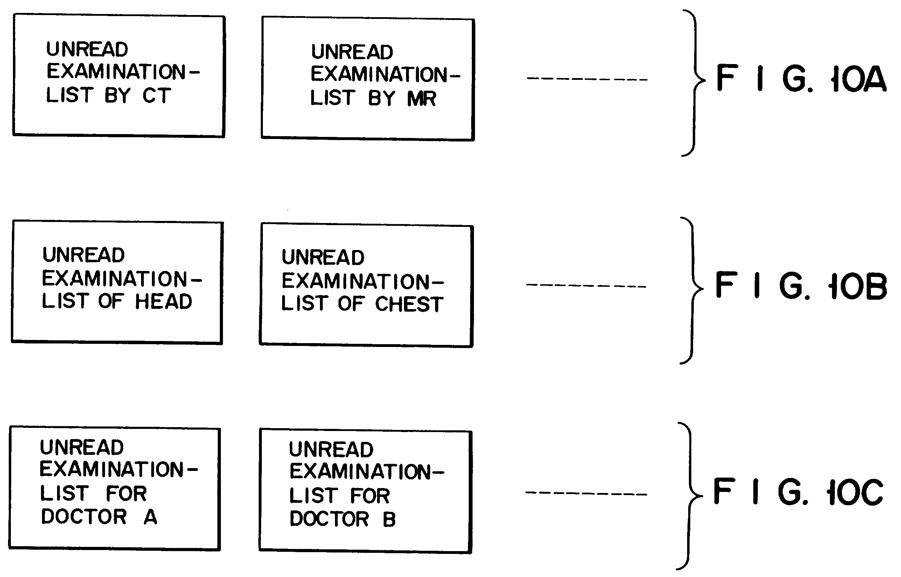

It is desirable that the number and classification of unread examination lists are set in accordance with the circumstances of each hospital. For instance, there may be an unread examination list for each modality (Fig. 10A), an unread examination list for each examination portion (Fig. 10B) and an unread examination list for each doctor (Fig. 10C). The present embodiment will be described with reference to the case where an unread examination list for each modality is prepared.

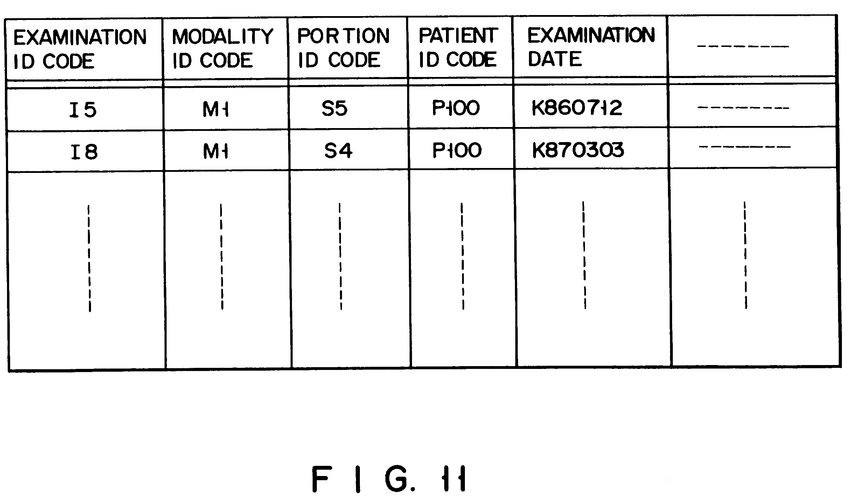

In step A4, image directory data of previous image data (hereinafter referred to as read image data) is retrieved using a patient ID code as retrieval data, and a patient previous examination list (hereinafter referred to as a read examination list) of the patient specified by the patient ID code is acquired (see Fig. 11). The read examination list is constituted by image addition data concerning the read image data.

In step A5, list data for the read examination list is arranged in accordance with a desired priority order.

Data concerning the priority order is set in advance in the DB. This priority order is set so as to arrange the list data in order, from the list data associated with image data having a high probability, to be referred by a doctor, a radiologist and the like. For instance, the priority order may be set as follows:
(1) Same patient, same examination portion and same modality.
(2) Same patient, same examination portion and other modality.

For the same priority order, the list data are arranged from a new examination date.

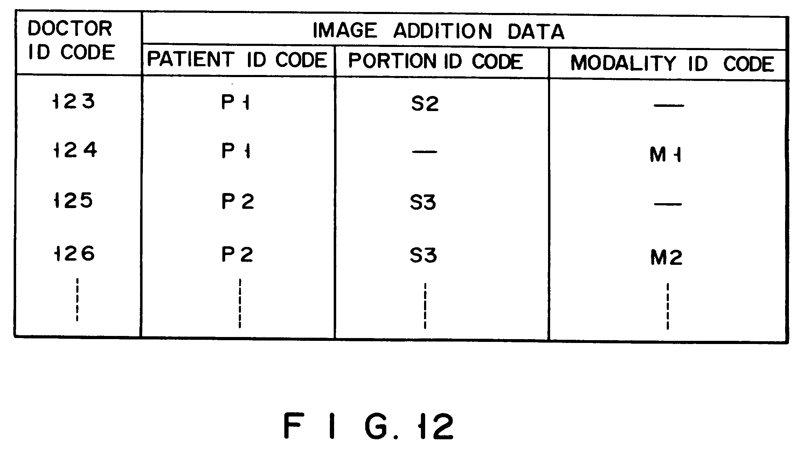

This priority order may differ depending on doctors and the like. As shown in Fig. 12, the priority order can be set by storing in advance data concerning the priority order in a magnetic card or the like which a doctor has, and reading out the data from the magnetic card as needed.

In step A6, address data of the read examination list is written on an unread examination list (see Fig. 9).

In step A7, image data and reports for a desired number of examinations are read out from an OD in accordance with the arranged order of the list data for the read examination list and are written in the MD.

Preparation of a reading operation in a data base will be completed through the above sequence.

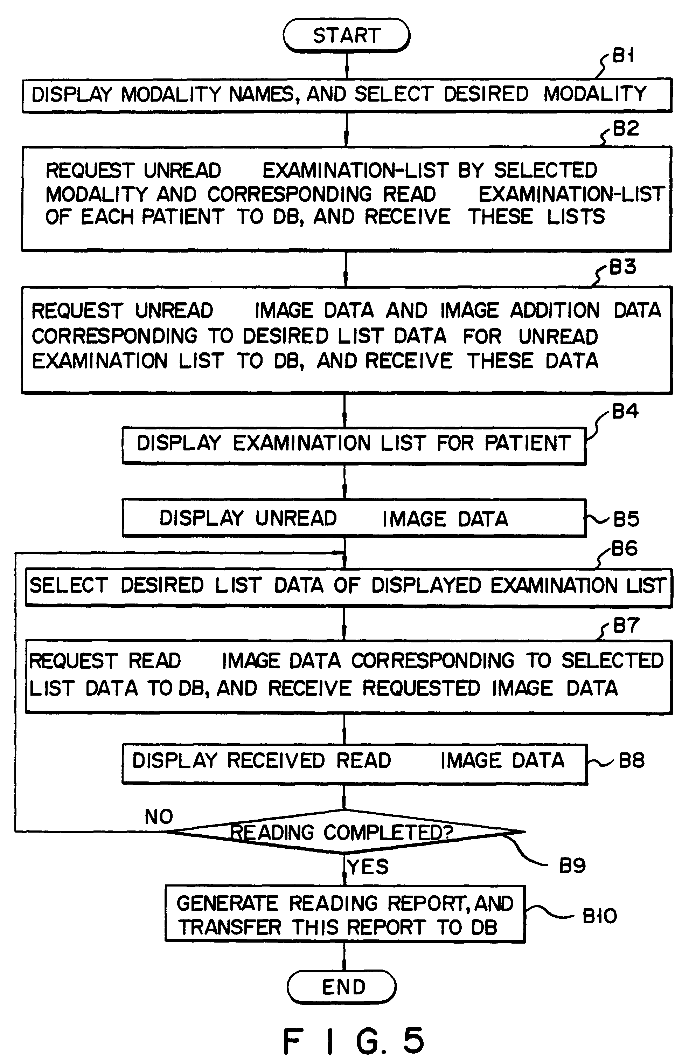

In the reading operation process in a work station as shown in Fig. 5, modality names are displayed and the desired modality associated with a doctor in charge is selected in step B1.

In step B2, an unread examination list by the selected modality and a corresponding examination list of each patient are requested to the DB and these lists are received from the DB.

In step B3, unread image data and image addition data corresponding to desired list data, for example, head list data, for the unread examination list are requested to the DB, and these data is received from the DB.

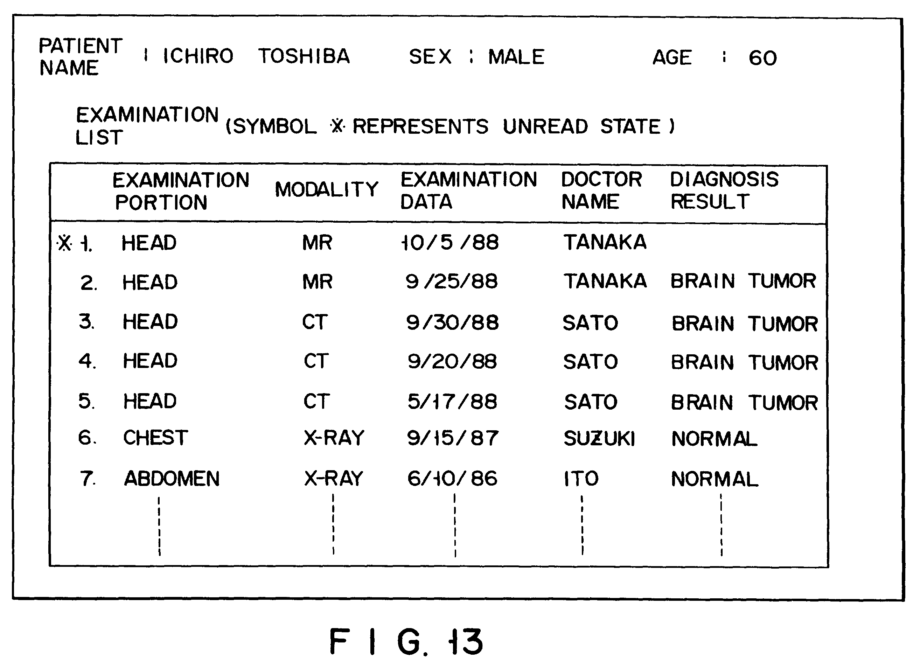

In step B4, an examination list for a desired patient is displayed as shown in Fig. 13.

In step B5, unread image data is displayed.

In step B6, the desired list data is selected from the displayed examination list.

In step B7, image data corresponding to the selected list data is requested to the DB and the requested image data is then received.

In step B8, the received read image data is displayed.

In step B9, it is determined whether or not a reading operation has been completed. If the reading operation has not been completed yet, the sequence following step B6 will be repeated. On the other hand, if the reading operation has been completed, a reading report is produced and transferred to the DB (step B10).

The reading operation process in the work station will be completed through the above sequence.

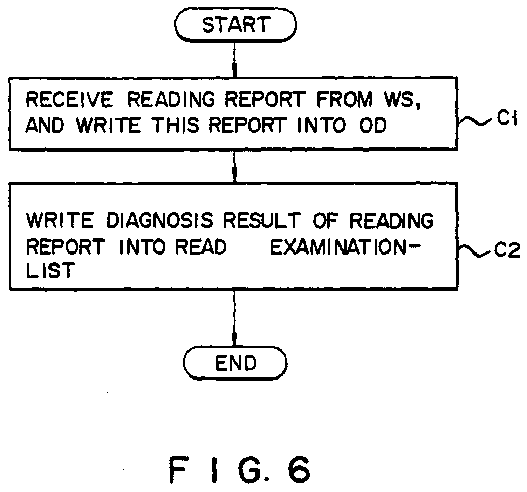

In the reading operation result storing process in the data base as shown in Fig. 6, the reading report transferred from the WS is received and stored in the OD in step C1.

In step C2, the diagnosis results of the reading report are written on the read examination list.

The process shown in Fig. 6 will be completed through the above two steps.

By executing the processes shown in Figs. 4 to 6 reading operation for a desired patient is completed.

According to this embodiment, as described above, an unread examination list and a read examination list are produced by specific image addition data (patient ID code), so that the examination efficiency can be improved.

In a data base, during production of the unread examination list and read examination list, image data and a report for each examination list, are transferred from an OD to an MD. The image data and report which a reference probability is high are not only transferred to the MD from the OD but are also transferred to the memory of the work station from the MD. Therefore, when the image data and report are requested, they can be displayed quickly, thus improving the reading operation efficiency.

Further, selection of a referred read image is facilitated by arranging list data of a read examination list in accordance with a desired priority order. This priority order can be set in advance in the data base or can be set for each doctor. Rearrangement of the list data can be performed in the data base as well as on the work station.

## Claims

1. A method for processing data in a medical information communication system, the method comprising the steps of:
storing image data and addition data indicative of plural identification parameters of the image data, the image data including previously read image data and unread image data;
generating a first list of a part of the unread image data which are selected in accordance with a desired one of the identification parameters and generating a second list of a part of the previously read image data which are selected in accordance with a desired one of the identification parameters;
rearranging the addition data included in the second list, thereby generating a third list of a part of the previously read image data; and
displaying said first and third lists and unread and previously read image data which are selected by using the first and third lists;
the method being characterized in that the addition data is rearranged according to a probability in which the previously read image data is referred to in reading the unread image data, thereby causing the third list to contain data which have a higher probability of being required.

2. An apparatus for processing data in a medical information communication system, the apparatus comprising:
storing means (30) for storing image data and addition data indicative of plural identification parameters of the image data, the image data including previously read image data and unread image data;
generating means (30) for generating a first list of a part of the unread image data which are selected in accordance with a desired one of the identification parameters and generating a second list of a part of the previously read image data which are selected in accordance with a desired one of the identification parameters;
rearranging means (25a ..., 25m) for rearranging the addition data included in the second list, thereby generating a third list of a part of the previously read image data; and
displaying means (25a, ..., 25m) for displaying said first and third lists and unread and previously read image data which are selected by using the first and the third lists;
the apparatus being characterized in that the rearrangement means causes the addition data to be rearranged according to a probability in which the previously read image data is referred to in reading the unread image data, thereby causing the third list to contain data which have a higher probability of being required.

3. The apparatus according to claim 2, further comprising plural modality means (10a,..., 10n) for acquiring the image data of a patient and the addition data.

4. The apparatus according to claim 2 or claim 3, wherein the storing means (30) includes an optical disk (39) and a magnetic disk (35, 36, 37, 38).

5. The apparatus according to claim 4, wherein said image data is transferred from the optical disk (39) to the magnetic disk (38) in accordance with the probability so that a part of the previously read image data which have a higher probability are stored in the magnetic disk (38).

6. A medical information communication system comprising a data processing apparatus according to claim 2 and further comprising:
data base means (30) for storing the image data and the addition data for generating the first and second lists;
work station means (25a, ..., 25m) for accessing the database means (30) to display the first and the unread image data,
said database means (30) generating the third list; and
the second list and the read image data are retrieved by said workstation means (25a, ..., 25m) from said database means (30) when the first list and the unread image data are displayed.

7. The apparatus according to claim 3, wherein
said generating means (30) generates the first list which is formed of the unread image data which are acquired by a given modality means and the second list which is formed of the read image data for a given patient; and
said rearranging means (25a, ...., 25m) rearranges the addition data included in the second list according to a name of said given modality means and an examination portion of the patient to be imaged.

8. The apparatus according to claim 2, wherein said rearranging means (25a, ...., 25m) comprises means for designating the probability used for generating the third list for every doctor.

9. The apparatus according to claim 8, wherein said designating means comprises a portable data storage card for storing the relationship between the doctor and the probability.

## Patentansprüche

1. Verfahren zur Datenverarbeitung in einem Übermittlungssystem für medizinische Informationen, wobei das Verfahren die Schritte umfaßt, daß man
- Bilddaten und zusätzliche Daten, die mehrere Identifikationsparameter der Bilddaten zeigen, speichert, wobei die Bilddaten vorher gelesene Bilddaten und ungelesene Bilddaten einschließen;
- eine erste Liste eines Teils der ungelesenen Bilddaten erzeugt, die in Übereinstimmung mit einem gewünschten der genannten Identilkationsparameter ausgewählt sind, und eine zweite Liste eines Teils der vorher gelesenen Bilddaten erzeugt, die in Übereinstimmung mit einem gewünschten der genannten Identifikationsparameter ausgewählt sind;
- die zusätzlichen Daten, die in der zweiten Liste eingeschlossen sind, umstellt, wodurch man eine dritte Liste eines Teils der vorher gelesenen Bilddaten erzeugt; und
- die erste und die dritte Liste sowie ungelesene und vorher gelesene Bilddaten anzeigt, die unter Verwendung der ersten und der dritten Liste ausgewählt werden;
wobei das Verfahren dadurch gekennzeichnet ist, daß die zusätzlichen Daten entsprechend einer Wahrscheinlichkeit umgestellt werden, gemäß der beim Lesen der ungelesenen Bilddaten auf die vorher gelesenen Bilddaten Bezug genommen wird, wodurch erreicht wird, daß die dritte Liste Daten enthält, die eine höhere Wahrscheinlichkeit aufweisen, benötigt zu werden.

2. Vorrichtung zur Datenverarbeitung in einem Übermittlungssystem für medizinische Informationen, umfassend
- eine Speicher-Einrichtung (30) zum Speichern von Bilddaten und zusätzlichen Daten, die mehrere Identilkationsparameter der Bilddaten anzeigen, wobei die Bilddaten vorher gelesene Bilddaten und ungelesene Bilddaten einschließen;
- eine Erzeugungs-Einrichtung (30) zum Erzeugen einer ersten Liste eines Teils der ungelesenen Bilddaten, die in Übereinstimmung mit einem gewünschten der genannten Identifikationsparameter ausgewählt werden, und zum Erzeugen einer zweiten Liste eines Teils der vorher gelesenen Bilddaten, die in Übereinstimmung mit einem gewünschten der vorgenannten Identifikationsparameter ausgewählt werden;
- Umstell-Einrichtungen (25a 25m) zum Umstellen der zusätzlichen Daten, die in der zweiten Liste eingeschlossen sind, wodurch eine dritte Liste eines Teils der vorher gelesenen Bilddaten erzeugt wird; und
- Anzeige-Einrichtungen (25a 25m) zum Anzeigen der ersten und der dritten Liste sowie ungelesener und vorher gelesener Bilddaten, die unter Verwendung der ersten und der dritten Liste ausgewählt werden;
wobei die Vorrichtung dadurch gekennzeichnet ist, daß die Umstell-Einrichtung dafür sorgt, daß die zusätzlichen Daten nach einer Wahrscheinlichkeit umgeordnet werden, mit der beim Lesen der ungelesenen Bilddaten auf die vorher gelesenen Bilddaten Bezug genommen wird, wodurch erreicht wird, daß die dritte Liste Daten enthält, die eine höhere Wahrscheinlichkeit aufweisen, benötigt zu werden.

3. Vorrichtung nach Anspruch 2, welche zusätzlich Einrichtungen (10a, ..., 10n) mit mehreren Betriebsarten zum Erfassen der Bilddaten eines Patienten und der zusätzlichen Daten umfaßt.

4. Vorrichtung nach Anspruch 2 oder Anspruch 3, worin die Speicher-Einrichtung (30) eine optische Platte (39) und eine Magnetplatte (35, 36, 37, 38) einschließt.

5. Vorrichtung nach Anspruch 4, worin die Bilddaten von der optischen Platte (39) auf die Magnetplatte (38) nach der Wahrscheinlichkeit übertragen werden, so daß ein Teil der vorher gelesenen Bilddaten, die eine höhere Wahrscheinlichkeit aufweisen, auf der Magnetplatte (38) gespeichert sind.

6. Übermittlungssystem für medizinische Informationen, umfassend eine Datenverarbeitungs-Vorrichtung nach Anspruch 2 und weiter umfassend
- eine Datenbank-Einrichtung (30) zum Speichern der Bilddaten und der zusätzlichen Daten zum Erzeugen der ersten und der zweiten Liste;
- Arbeitsstations-Einrichtungen (25a, ..., 25m) zum Zugriff auf die Datenbank-Einrichtung (30) und Anzeigen der ersten und der ungelesenen Bilddaten, wobei die Datenbank-Einrichtung (30) die dritte Liste erzeugt; und die zweite Liste und die gelesenen Bilddaten durch die Arbeitsplatz-Einrichtungen (25a, ..., 25m) von der Datenbank-Einrichtung (30) zurückgeladen werden, wenn die erste Liste und die ungelesenen Bilddaten angezeigt werden.

7. Vorrichtung nach Anspruch 3, worin die Erzeugungs-Einrichtung (30) die erste Liste, die aus den ungelesenen Bilddaten gebildet wird, die durch eine gegebene Betriebsarten-Einrichtung erfaßt werden, und die zweite Liste, die aus den gelesenen Bilddaten für einen gegebenen Patienten gebildet wird, erzeugt; und
- die Umstell-Einrichtung (25a, ..., 25m) die zusätzlichen Daten, die in die zweite Liste eingeschlossen sind, nach dem Namen einer gegebenen Betriebsarten-Einrichtung und einem Untersuchungsabschnitt des abzubildenden Patienten umstellt.

8. Vorrichtung nach Anspruch 2, worin die Umstell-Einrichtung (25a 25m) eine Einrichtung zum Bezeichnen der Wahrscheinlichkeit umfaßt, die zum Erzeugen der dritten Liste für jeden Arzt verwendet wird.

9. Vorrichtung nach Anspruch 8, worin die Bezeichnungs-Einrichtung eine tragbare Datenspeicher-Karte zum Speichern der Beziehung zwischen dem Arzt und der Wahrscheinlichkeit umfaßt.

## Revendications

1. Méthode pour le traitement des données dans un système de communication d'informations médicales, la méthode comprenant les étapes de:
stockage de données d'image et de données additionnelles indicatives de plusieurs paramètres d'identification des données d'image, les données d'image incluant des données d'image lues précédemment et des données d'image non lues;
génération d'une première liste d'une partie des données d'image non lues qui sont sélectionnées conformément à l'un voulu des paramètres d'identification et génération d'une deuxième liste d'une partie des données d'image lues précédemment qui sont sélectionnées conformément à l'un voulu des paramètres d'identification;
reclassement des données additionnelles incluses dans la deuxième liste, pour générer ainsi une troisième liste d'une partie des données d'image lues précédemment; et,
visualisation desdites première et troisième listes et de données d'image non lues et lues précédemment qui sont sélectionnées par l'utilisation des première et troisième listes;
la méthode étant caractérisée en ce que les données additionnelles sont reclassées selon la probabilité qu'on se réfère aux données d'image lues précédemment dans la lecture des données d'image non lues, la troisième liste contenant ainsi des données qui ont une plus haute probabilité d'être demandées.

2. Appareil pour le traitement des données dans un système de communication d'informations médicales, l'appareil comprenant:
des moyens de stockage (30) pour stocker des données d'image et des données additionnelles indicatives de plusieurs paramètres d'identification des données d'image, les données d'image incluant des données d'image lues précédemment et des données d'image non lues;
des moyens de génération (30) pour générer une première liste d'une partie des données d'image non lues qui sont sélectionnées conformément à l'un voulu des paramètres d'identification et générer une deuxième liste d'une partie des données d'image lues précédemment qui sont sélectionnées conformément à l'un voulu des paramètres d'identification;
des moyens de reclassement (25a,...,25m) pour reclasser les données additionnelles incluses dans la deuxième liste, pour générer ainsi une troisième liste d'une partie des données d'image lues précédemment; et,
des moyens de visualisation (25a,...,25m) pour visualiser lesdites première et troisième listes et des données d'image non lues et lues précédemment qui sont sélectionnées par l'utilisation des première et troisième listes;
l'appareil étant caractérisé en ce que les moyens de reclassement font en sorte que les données additionnelles soient reclassées selon la probabilité qu'on se réfère aux données d'image lues précédemment dans la lecture des données d'image non lues, la troisième liste contenant ainsi des données qui ont une plus haute probabilité d'être demandées.

3. Appareil selon la revendication 2, comprenant en outre une pluralité de moyens de modalité (10a,...,10n) pour acquérir les données d'image d'un patient et les données additionnelles.

4. Appareil selon la revendication 2 ou la revendication 3, dans lequel les moyens de stockage (30) comprennent un disque optique (39) et un disque magnétique (35,36,37,38).

5. Appareil selon la revendication 4, dans lequel lesdites données d'image sont transférées du disque optique (39) au disque magnétique (38) selon la probabilité qu'une partie des données d'image lues précédemment qui ont une plus haute probabilité soient stockées sur le disque magnétique (38).

6. Système de communication d'informations médicales comprenant un appareil de traitement de données selon la revendication 2 et comprenant en outre:
des moyens de base de données (30) pour stocker les données d'image et les données additionnelles pour la génération des première et deuxième listes;
des moyens de poste de travail (25a,...,25m) pour accéder aux moyens de base de données (30) pour la visualisation de la première liste et des données d'image non lues,
lesdits moyens de base de données (30) générant la troisième liste; et,
la deuxième liste et les données d'image lues sont recherchées par lesdits moyens de poste de travail (25a,...,25m) dans lesdits moyens de base de données (30) quand la première liste et les données d'image non lues sont visualisées.

7. Appareil selon la revendication 3, dans lequel lesdits moyens de génération (30) génèrent la première liste qui est constituée des données d'image non lues qui sont acquises par un moyen de modalité donné et la deuxième liste qui est constituée des données d'image lues pour un patient donné; et,
lesdits moyens de reclassement (25a 25m) reclassent les données additionnelles incluses dans la deuxième liste selon le nom dudit moyen de modalité donné et une partie d'examen du patient dont une image doit être formée.

8. Appareil selon la revendication 2, dans lequel lesdits moyens de reclassement (25a,...,25m) comprennent un moyen pour indiquer la probabilité utilisée pour générer la troisième liste pour chaque médecin.

9. Appareil selon la revendication 8, dans lequel ledit moyen d'indication comprend une carte portable de stockage de données pour stocker la relation entre le médecin et la probabilité.
